Europäisches Patentamt

**European Patent Office**

**Office européen des brevets**

(11) Veröffentlichungsnummer: **0 236 996**
B1

(12) EUROPÄISCHE PATENTSCHRIFT

(45) Veröffentlichungstag der Patentschrift:
31.10.90

(51) Int. Cl.⁵: **C07C 249/14**

(21) Anmeldenummer: **87103287.6**

(22) Anmeldetag: **07.03.87**

(54) **Verfahren zur Reinigung von Ammoniumsulfat enthaltendem Cyclohexanonoxim.**

(30) Priorität: **11.03.86 DE 3607997**

(43) Veröffentlichungstag der Anmeldung:
**16.09.87 Patentblatt 87/38**

(45) Bekanntmachung des Hinweises auf die Patenterteilung:
**31.10.90 Patentblatt 90/44**

(84) Benannte Vertragsstaaten:
**BE CH DE FR GB IT LI NL**

(56) Entgegenhaltungen:
**DE-A- 1 768 210**

**Fachlexikon ABC Chemie, Verlag Harri Deutsch, Frankfurt/Main, Band 1, 2-Auflage 1979, Seiten 586-89 Houben-Weyl, Methoden der organischen Chemie, Georg, Thieme Verlag Stuttgart, Band I/1, 1958 Seiten 549, 556-57, 559**

(73) Patentinhaber: **BASF Aktiengesellschaft, Carl-Bosch-Strasse 38, D-6700 Ludwigshafen(DE)**

(72) Erfinder: **Fuchs, Hugo, Dr., Egellstrasse 28, D-6700 Ludwigshafen(DE)**

## Beschreibung

Cyclohexanonoxim wird in großem technischen Maßstab durch Umsetzen von Cyclohexanon mit wäßriger Hydroxylammoniumsulfatlösung bei einer Temperatur über dem Schmelzpunkt des Cyclohexanonoxims hergestellt. Die so anfallende Schmelze von Cyclohexanonoxim enthält noch ca. 5-8 Gew.-% Wasser und zusätzlich Ammoniumsulfat das teils aus der Hydroxylaminsynthese, teils aus der Neutralisation während der Oximierung anfällt. Diese Mengen an Ammoniumsulfat neigen dazu auszukristallisieren, wenn der Wassergehalt des Cyclohexanoxims erniedrigt wird, um bei der Herstellung von Caprolactam einen möglichst niedrigen Verbrauch an Oleum zu erzielen. Dies führt zu Verstopfungen der Düsen bei der Weiterverarbeitung zu Caprolactam. Entsprechend dem in der DE-PS 20 29 114 beschriebenen Verfahren wird bei der Herstellung von Cyclohexanonoxim ein mit Wasser nicht mischbares Lösungsmittel mitverwendet. Die Wiedergewinnung des Lösungsmittels ist jedoch technisch aufwendig, abgesehen von den unvermeidlich entstehenden Lösungsmittelverlusten. Nach einem anderen in der DE-AS 17 68 210 beschriebenen Verfahren soll Cyclohexanonoxim mit entsalztem Wasser ausgewaschen werden. Abgesehen davon, daß der Wassergehalt ansteigt und zu einem erhöhten Verbrauch an Oleum führt, treten Trennprobleme bei der Abtrennung des Cyclohexanonoxims vom entsalztem Wasser auf.

Es war deshalb die technische Aufgabe gestellt, Cyclohexanonoxim mit einem geringen Gehalt an Ammoniumsulfat zur Verfügung zu stellen, das ohne Salzausscheidungen zu Caprolactam weiterverarbeitet werden kann.

Diese Aufgabe wird gelöst in einem Verfahren zur Reinigung von Ammoniumsulfat enthaltendem Cyclohexanonoxim, wobei man Cyclohexanonoxim in schmelzflüssigem Zustand bei einer Temperatur von 70 bis 95°C über einen sauren Ionenaustauscher und einen basischen Ionenaustauscher leitet.

Das neue Verfahren hat den Vorteil, daß Ammoniumsulfat auf einfache Weise aus dem Cyclohexanonoxim entfernt wird, ohne daß der Wassergehalt ansteigt oder Trennprobleme auftreten. Ferner hat das neue Verfahren den Vorteil, daß man Cyclohexanonoxim erhält, das bei der Weiterverwendung bei der Herstellung von Caprolactam nicht zu Verstopfungen der Zufuhrdüsen führt. Ferner hat das neue Verfahren den Vorteil, daß sich so hergestelltes Cyclohexanonoxim ohne Salzausscheidungen weiter entwässern läßt.

Erfindungsgemäß geht man von Ammoniumsulfat enthaltendem Cyclohexanonoxim aus. In der Regel enthält solches Cyclohexanonoxim 5 bis 8 Gew.-% Wasser und 10 bis 1000 mg Ammoniumsulfat je kg Cyclohexanonoxim. Neben Cyclohexanonoxim können noch geringe Mengen an Cyclohexanon z.B. 10 bis 1000 mg je kg enthalten sein. Solches Cyclohexanonoxim erhält man beispielsweise nach einem in der DE-P S 1 205 966 beschriebenen Verfahren, ggf. durch zusätzliche Partiellentwässerung mit konzentrierter Ammoniumsulfatlösung entsprechend DE-PS 21 38 930.

Cyclohexanonoxim wird erfindungsgemäß in schmelzflüssigem Zustand über einen sauren und einen basischen Ionenaustauscher geleitet, wobei man das Cyclohexanonoxim auf einer Temperatur von 70 bis 95°C hält. Vorzugsweise wendet man schwach saure Ionenaustauscher, z.B. vernetzte Polymere mit Carbonsäuregruppen, an. Solche sind beispielsweise aufgebaut aus vernetztem Polyacrylat das Carbonsäuregruppen enthält. Desgleichen verwendet man vorteilhaft schwach basische Ionenaustauscher. Geeignete Ionenaustauscher sind beispielsweise aufgebaut aus vernetztem Polystyrol, das primäre, sekundäre oder tertiäre Aminogruppen enthält. Es hat sich besonders bewährt, wenn man geschmolzenes Cyclohexanonoxim zunächst über einen schwach sauren Ionenaustauscher leitet und anschließend über einen schwach basischen Ionenaustauscher. Es ist aber auch eine Kombination schwach basisch-schwach saurerschwach basische Ionenaustauscher möglich.

Nach Erschöpfung der Ionenaustauscher werden diese in üblicher Weise z.B. mit Säuren wie Schwefelsäure bzw. Basen wie Ammoniakwasser oder wäßriger Natronlauge reaktiviert.

Das so erhaltene noch Restwasser z.B. 5 bis 8 Gew.% Wasser enthaltende Cyclohexanonoxim kann ohne Störungen weiter entwässert werden, z.B. auf einen Wassergehalt von 3 Gew.-% durch Behandeln mit heißen Inertgasen in der Schmelze entsprechend den in der DE-AS 10 34 629 beschriebenen Verfahren.

Nach dem erfindungsgemäßen Verfahren ist Cyclohexanonoxim mit einem Gehalt an Ammoniumsulfat von < 5 mg/kg erhältlich.

Das Verfahren nach der Erfindung sei in folgendem Beispiel veranschaulicht.

### Beispiel

In einem zylindrischen Glasrohr von Durchmesser 30 mm, welches mit einem Heizmantel versehen ist und am unteren Ende eine Fritte enthält, werden 75 ml eines schwach sauren Ionenaustauschers aufgebaut aus Polyacrylat mit Carboxylgruppen eingefüllt. Eine zweite Ionenaustauschersäule des gleichen Durchmessers und ebenfalls mit einem Heizmantel versehen, gefüllt mit 100 ml eines schwach basischen Ionenaustauschers, aufgebaut aus vernetztem Polystyrol mit tert. Aminogruppen, wird der ersten Säule nachgeschaltet. Beide Ionenaustauschersäulen werden auf eine Temperatur von 80°C gebracht. Anschließend werden 300 g/h geschmolzenes Cyclohexanonoxim mit einem Wassergehalt von 5 % (m/m), einem Restcyclohexanongehalt von 50 mg/kg, sowie einem Ammoniumsulfatgehalt von 80 mg/kg über die Ionenaustauschersäulen geleitet. Das aus der Austauschersäule ablaufende Cyclohexanonoxim enthält 5 % (m/m) Wasser, 55 mg/kg Cyclohexanon und einen Ammonsulfatgehalt von nur noch 3 mg/kg. Dieses Oxim läßt sich nun durch Behandlung mit Luft oder $N_2$ bis auf den gewünschten Wassergehalt einstellen, ohne daß Störungen durch ausfallendes Ammoniumsulfat auftreten.

**Patentansprüche**

1. Verfahren zur Reinigung von Ammoniumsulfat enthaltendem Cyclohexanonoxim, dadurch gekennzeichnet, daß man Cyclohexanonoxim in schmelzflüssigem Zustand bei einer Temperatur von 70 bis 95°C über einen sauren und einen basischen Ionenaustauscher leitet.

2. Verfahren nach Anspruch 1, dadurch gekennzeichnet, daß man schwach saure und schwach basische Ionenaustauscher anwendet.

3. Verfahren nach den Ansprüchen 1 und 2, dadurch gekennzeichnet, daß man das schmelzflüssige Cyclohexanonoxim zunächst über einen schwach sauren Ionenaustauscher und dann über einen schwach basischen Ionenaustauscher leitet.

4. Verfahren nach den Ansprüchen 1 bis 3, dadurch gekennzeichnet, daß man 5 bis 8 Gew.-% Wasser sowie 10 bis 1000 mg Ammoniumsulfat je kg enthaltendes Cyclohexanonoxim verwendet.

**Claims**

1. A process for the purification of cyclohexanone oxime containing ammonium sulfate, wherein cyclohexanone oxime in the molten state is passed at from 70 to 95°C over an acidic ion exchanger and a basic ion exchanger.

2. A process as claimed in claim 1, wherein weakly acidic and weakly basic ion exchangers are used.

3. A process as claimed in claim 1 or 2, wherein the molten cyclohexanone oxime is passed first over a weakly acidic ion exchanger and then over a weakly basic ion exchanger.

4. A process as claimed in any of claims 1 to 3, wherein cyclohexanone oxime containing from 5 to 8% by weight of water and from 10 to 1000 mg of ammonium sulfate per kg is used.

**Revendications**

1. Procédé de purification de cyclohexanone-oxime, contenant du sulfate d'ammonium, caractérisé en ce qu'on fait passer la cyclohexanone-oxime à l'état fondu à une température de 70 à 95°C sur un échangeur d'ions acide et sur un échangeur d'ions basique.

2. Procédé selon la revendication 1, caractérisé en ce qu'on utilise des échangeurs d'ions fortement acide et fortement basique.

3. Procédé selon les revendications 1 et 2, caractérisé en ce qu'on fait passer la cyclohexanone-oxime fondue d'abord sur un échangeur d'ions fortement acide puis sur un échangeur d'ions fortement basique.

4. Procédé selon les revendications 1 à 3, caractérisé en ce qu'on utilise de la cyclohexanone-oxime contenant 5 à 8% en poids d'eau ainsi que 10 à 1000 mg de sulfate d'ammonium par kg de cyclohexanone-oxime.